# EUROPEAN PATENT APPLICATION

(11) **EP 4 502 561 A1**
(43) Date of publication of application: **05.02.2025**
(21) Application number: 24192019.8
(22) Date of filing: 31.07.2024
(51) Int. Cl.: G01K 1/02, G01K 7/02, H01B 7/08, A61B 5/01, A61M 25/00, G01K 13/20

(54) **RIBBON CABLE WITH SENSOR FORMED THEREON AND METHOD OF MANUFACTURE**

(30) Priority: 02.08.2023 US 202318364019
(71) Applicant: TE Connectivity Solutions GmbH, 8200 Schaffhausen (CH)
(72) Inventor: WANG, Jian, Fremont, 94555 (US); SHOGREN, Jon, Andover, 55304 (US); MORALES, Miguel, Fremont, 94555 (US); BHARADWAJ, Lavanya, San Ramon, 94583 (US); WU, Yiliang, San Ramon, 94582 (US)
(74) Representative: Ashton, Gareth Mark

(57) **Abstract**

A ribbon cable sensor assembly (2) having a ribbon cable (10) with respective individual wires (14). At least one sensor terminating area (20) is positioned on the ribbon cable. The at least one sensor terminating area has insulation (24) removed from respective individual wires (14) to provide exposed individual conductors. Conductive material (26) is applied to respective exposed individual conductors in the at least one sensor terminating area to form at least one sensor (12). Nonconductive material (30) may be applied to sensor terminating area on top of the conductive material.

## Description

### FIELD OF THE INVENTION

The invention relates to a ribbon cable with one or more sensors formed or fabricated thereon and the method of manufacture thereof.

### BACKGROUND OF THE INVENTION

Electrical components, such as sensors, can be attached to wires or cable. One such example is the fabrication of micro-thermocouple (MTC) sensors with a two-wire cable used for medical catheters. In such application, the ends of the wires are manually stripped, at the stripped ends of the cable a heat shrink member or sock may then be applied for electrical insulation.

While the method described above is sufficient for a termination of a single sensor, multiple sensors cannot be fabricated on the same cable at different locations. The use of multiple sensors which are spaced at different locations can be beneficial in many applications. One such application is MTC sensors at spaced locations in a catheter for multiple point temperature measurements. Currently, the spaced sensors are terminated on individual cables, bundled together and inserted into the catheter. This requires extra space inside the catheter and increases customer labor costs.

It would, therefore, be beneficial to provide a ribbon cable system with one or more sensors and a method of manufacturing the same. In particular, it would be beneficial to provide a system in which one or more sensors could be fabricated on a single ribbon cable or the like by means to minimize space requirement and which could be fabricated by an automated process to reduce manufacturing and labor costs.

### SUMMARY OF THE INVENTION

The following provides a summary of certain illustrative embodiments of the present invention. This summary is not an extensive overview and is not intended to identify key or critical aspects or elements of the present invention or to delineate its scope.

An aspect of the invention is defined in the appended claim 1. Printed electronics technology may be applied for the fabrication of micro-thermocouple (MTC) ribbon sensors. This may include the use of dispensing technology for precisely applying solder paste or silver ink to form the micro-junctions, and the use of dispensing technology for applying coating to encapsulate the micro-junctions for the electrical insulation. Jet dispensing is one such dispensing technology which can be used for low volume, high speed applications with high consistency.

An embodiment is directed to a ribbon cable sensor assembly having a ribbon cable with respective paired conductors. At least one sensor terminating area is positioned on the ribbon cable. The at least one sensor terminating area has electrical insulation removed from respective individual wires of the respective paired conductors to provide exposed individual conductors. Conductive material is applied to respective exposed individual conductors in the at least one sensor terminating area to form at least one sensor. Nonconductive material may be applied to sensor terminating area on top of the conductive material.

In various embodiments, the at least one sensor terminating area are multiple sensor terminating areas spaced apart along a longitudinal axis of the ribbon cable and the at least one sensor are multiple sensors positioned in the multiple sensor terminating areas to allow the multiple sensors to sense different regions along the ribbon cable.

An embodiment is directed to a method of manufacturing a ribbon cable sensor assembly with one or more sensors. The method includes: exposing individual conductors of the ribbon cable in a defined area by removing electrical insulation in the defined areas; applying conductive ink in the defined area on the exposed individual conductors of the cable to form a sensor; and curing the conductive ink.

Additional features and aspects of the present invention will become apparent to those of ordinary skill in the art upon reading and understanding the following detailed description of the exemplary embodiments. As will be appreciated by the skilled artisan, further embodiments of the invention are possible without departing from the scope and spirit of the invention. Accordingly, the drawings and associated descriptions are to be regarded as illustrative and not restrictive in nature.

### BRIEF DESCRIPTION OF THE DRAWINGS

The accompanying drawings, which are incorporated into and form a part of the specification, schematically illustrate one or more exemplary embodiments of the invention and, together with the general description given above and detailed description given below, serve to explain the principles of the invention.
FIG. 1 is a perspective view of a ribbon cable with multiple sensors at different locations of the cable.
FIG. 2 is a diagrammatic view of a portion of the ribbon cable prior to fabricating a sensor thereon.
FIG. 3 is a diagrammatic view of the ribbon cable of FIG. 2 provided proximate a laser, the ribbon cable has a portion of the insulation removed to expose individual conductors, the ribbon cable is provided proximate a printer.
FIG. 4 is a diagrammatic view of the ribbon cable provided proximate a first printer, the ribbon cable has conductive ink deposited on the exposed wires to form a sensor.
FIG. 5 is a diagrammatic view of the ribbon cable provided proximate a second printer, the ribbon cable has the conductive ink of FIG. 4 cured and insulative material deposited on the cured conductive ink and the exposed wires.
FIG. 6 is a diagrammatic view of the ribbon cable assembly positioned in a catheter.
FIG. 7 is a flowchart illustrating an illustrative method of manufacturing the ribbon cable assembly with multiple sensors shown in FIG. 1.
FIG. 8 is a perspective view of an alternative embodiment of a ribbon cable with a sensor fabricated at an end of the ribbon cable.

### DETAILED DESCRIPTION OF THE INVENTION

The description of illustrative embodiments according to principles of the present invention is intended to be read in connection with the accompanying drawings, which are to be considered part of the entire written description. In the description of embodiments of the invention disclosed herein, any reference to direction or orientation is merely intended for convenience of description and is not intended in any way to limit the scope of the present invention. Relative terms such as "lower," "upper," "horizontal," "vertical," "above," "below," "up," "down," "top" and "bottom" as well as derivative thereof (e.g., "horizontally," "downwardly," "upwardly," etc.) should be construed to refer to the orientation as then described or as shown in the drawing under discussion. These relative terms are for convenience of description only and do not require that the apparatus be constructed or operated in a particular orientation unless explicitly indicated as such. Terms such as "attached," "affixed," "connected," "coupled," "interconnected," and similar refer to a relationship wherein structures are secured or attached to one another either directly or indirectly through intervening structures, as well as both movable or rigid attachments or relationships, unless expressly described otherwise. Moreover, the features and benefits of the invention are illustrated by reference to the preferred embodiments. Accordingly, the invention expressly should not be limited to such preferred embodiments illustrating some possible non-limiting combination of features that may exist alone or in other combinations of features, the scope of the invention being defined by the claims appended hereto.

Exemplary embodiments of the present invention are now described with reference to the Figures. Reference numerals are used throughout the detailed description to refer to the various elements and structures. Although the following detailed description contains many specifics for the purposes of illustration, a person of ordinary skill in the art will appreciate that many variations and alterations to the following details are within the scope of the invention. Accordingly, the following embodiments of the invention are set forth without any loss of generality to, and without imposing limitations upon, the claimed invention.

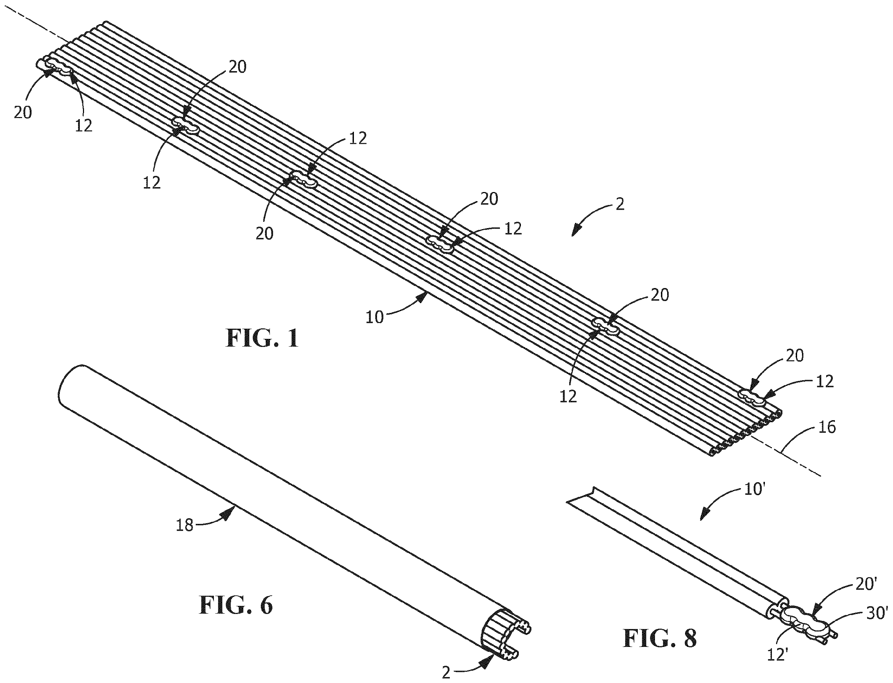

A multi-conductor ribbon cable sensor assembly 2 is shown in FIG. 1. The multi-conductor ribbon cable sensor 2 is meant to be illustrative, as different configurations, different sizes and different types of cable can be used.

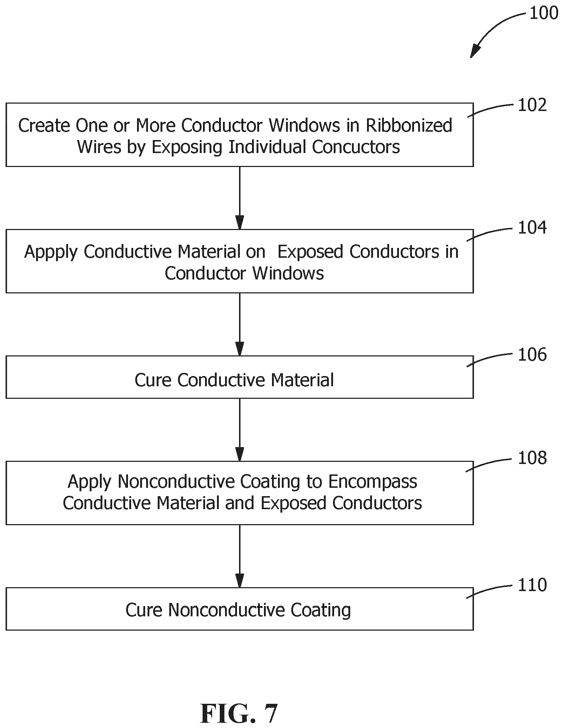

In general, the ribbon cable has ribbonized cable or ribbon cable 10 with one or more sensors 12 formed or fabricated to individual conductors or wires 14 of paired conductors of the ribbon cable 10 at sensor terminating areas 20. In the illustrative embodiment shown, six sensors 12 are electrically and mechanically formed on respective pairs of the individual wires 14. The sensors 12 are spaced apart along a longitudinal axis 16 of the ribbon cable 10 to allow the sensors 12 to be used to sense different regions or areas. For example, the ribbon cable 10 may be positioned in a catheter 18 (FIG. 7) and the sensors 12 may be temperature sensors to allow for temperatures to be monitored at different and multiple locations along the catheter 18.

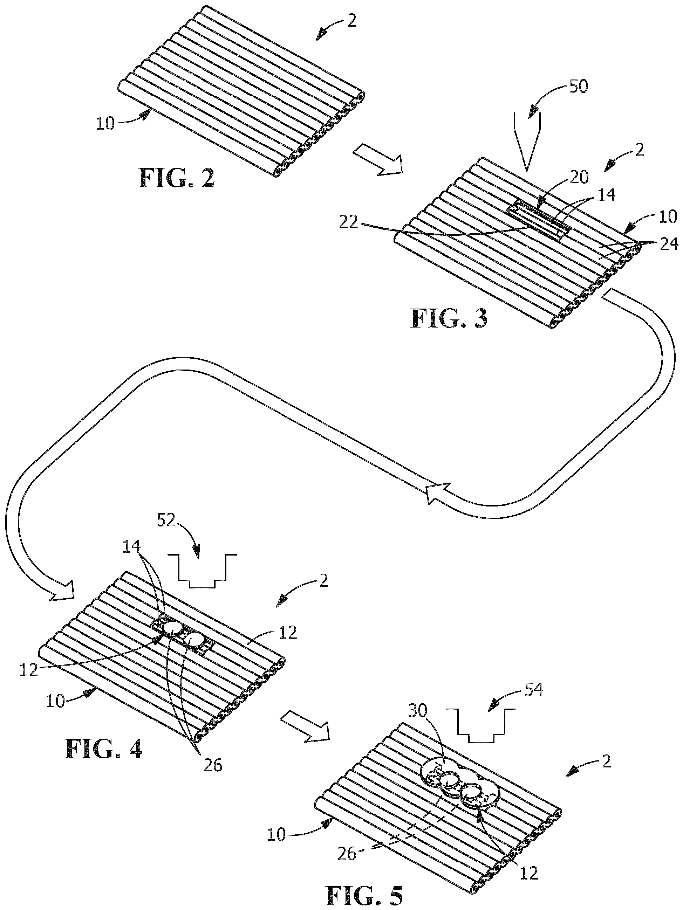

Each sensor terminating zone has a conductor window 22 (FIG. 3) in which the insulation 24 of two respective individual wires 14 have been removed around individual wires 14 of the paired conductors. Conductive material 26 (FIG. 4) is applied to the exposed conductors 14. The conductive material 26 is provided in mechanical and electrical engagement with the respective wires 14 that is positioned in the respective sensor terminating area 20. The conductive material 26 cooperates with the exposed conductors to form the sensor 12. A nonconductive or electrically insulative material 30 (FIG. 5) extends over the conductive material 26 and the exposed conductors 14 to seal or cover the conductive material 26 and the exposed conductors 14 in the conductor window 22 to protect and insulate the sensor 12 formed by the conductive material 26 and the exposed conductors 14.

Referring to FIG. 7, the general method 100 of creating the sensor terminating areas 20 in the ribbon cable 10 is illustrated in a flow chart format. Initially the conductor windows 22 are created in the ribbon cable 10, removing the insulation around respective individual wires 14 of the ribbon cable 10, as represented in step 102. The conductive material 26 is applied to the exposed conductors 14 in the conductive windows 22 to form the sensor 12, as represented in step 104. The conductive material 26 is then cured to provide a mechanical and electrical connection between the exposed conductors 14 in the conductor windows 22, as represented in step 106. The nonconductive or insulative material 30 is then applied to over the conductive material 26 and the conductor window 22, as represented in step 108. The nonconductive or insulative material 30 is then cured to provide a seal or cover to protect and insulated the sensor 12 formed by the conductive material 26 and the exposed conductors 14, as represented in step 110.

In the illustrative embodiment shown in FIG. 3, the conductor window 22 is formed by a laser 50 to precisely remove the insulation around two respective individual wires 14 of the ribbon cable 10. The two wires 14 are immediately adjacent one another, aligned side-by-side in parallel, and constitute a pair of conductors. This type of laser ablation can be used in automated processes to increase the production of the finished multi-conductor ribbon cable sensor assembly 2. Other methods and devices for removing the insulation around respective individual wires 14 of the ribbon cable 10 to create the conductor window 22 may be used.

In the illustrative embodiment shown, the conductor windows 22 are approximately 150 microns by approximately 400 microns. Other dimensions for the conductor windows 22 may be used based on the type of sensors to be used and the final use of the multi-conductor ribbon cable sensor assembly 2.

With the conductor window 22 formed in the ribbon cable 10, the ribbon cable 10 is moved into position relative to a dispensing nozzle 52 of a print head, as shown in FIG. 4. The dispensing nozzle 52 is aligned with the center of the exposed pairs of conductors 14 in the respective conductor window 22.

The use of a printed electronics printer allows for the conductive material 26 to be precisely applied to the exposed pairs of conductors 14 in the respective conductor window 22. In particular, the conductive material 26 can be applied such that the conductive material 26 shorts the exposed pairs of conductors 14 to form the micro-junctions of the sensor 12.

Various printing technologies can be used to apply the conductive material 26 through dispensing nozzle 52, including, but not limited to, inkjet printing, contact extrusion dispensing, jet dispensing. In the illustrative embodiment shown, the dispensing nozzle 52 is part of a jet dispensing system. With jet dispensing, the dispensing nozzle or jet 52 hovers over and shoots the conductive material 26 over the conductor window 22 without ever touching the exposed conductors 14. Therefore, the z-axis motion of dispensing nozzle 52 can be eliminated for printing on uneven or irregularly shaped surfaces of the ribbon cable 10. In addition, the dispensing nozzle or jet 52 produces dots of extremely small size (for example, less than approximately 150 um), low volume (for example, approximately 0.5 nL), high speed (for example, up to approximately 1000 dots/s) and high consistency. While various jet dispensing nozzles 52 may be used, one example is the MDS 3280 dispensing system, available from VERMES Microdispensing GmbH. The dispensing is based on piezoelectric actuator with a unique bayonet fluid box body, which is more suitable for volume production.

The dispensing nozzle 52 may have different configurations and different sizes of an inner channel of the nozzle insert which can affect the dispensing volume. In an illustrative embodiment, the nozzle insert has a conical inner channel, causing the conductive material 26 to accelerate while exiting the dispensing nozzle 52 resulting in less buildup of the conductive material 26 on the dispensing nozzle 52. The size of the tappet tip has an influence on the dispensing energy of the conductive material 26. In one illustrative embodiment, the nozzle insert has an approximately 50 µm channel and the tappet tip has a radius of approximately 5 µm to facilitate the dispensing of the conductive material 26.

In the embodiment shown in FIG. 4, two droplets of conductive material 26 are dispensed from the dispensing nozzle 52 onto the exposed pairs of wires 14 in the respective conductor window 22. In other embodiments, one, three or more than three droplets may be applied. The droplets may be as small as approximately 0.5 nL which leads to drop size of approximately 200 microns to approximately 400 microns in diameter when applied to the exposed pairs of conductors 14. The droplets may be applied at a speed up to approximately 500 dots/s, with no need of z-axis motion for conformal printing. In other embodiments, the diameter of the dispensed conductive material 26 can be down to approximately 150 microns.

In the illustrative embodiment shown, the conductive material 26 can be, but is not limited to solder paste, silver ink and gold ink. One example is a nano-silver ink. The resistivity of the cured silver ink may vary, but in this illustrative embodiment, the resistivity is between approximately 0.01 mohm·cm and approximately 5 mohm·cm. The silver ink has approximately 0.3 micron or less diameter silver particles. An illustrative example of the silver ink is Metalon^{®} HPS-FG77 silver ink, available from NovaCentrix Corporation. The silver ink may be diluted between approximately 0.5% to approximately 3% solvent, such as, but not limited to, butyl carbitol, to improve the ink self-leveling properties. The thickness of the cured silver trace was measured using 3D optical profiler. The thickness of the silver ink is between approximately 20 microns to approximately 30 microns.

In one illustrative embodiment, the jet dispensing process setting for the conductive material 26 has: a rise time of from approximately 0.2 ms to approximately 0.3 ms; a fall time of from approximately 0.1 ms to approximately 0.25 ms; and open time of from approximately 0.2 ms to approximately 0.4 ms; a needle lift of from approximately 68% to approximately 72%; a nozzle height of from approximately 1.8 mm to approximately 2 mm; a pressure of from approximately 0.07 MPa to approximately 0.1 MPa; and a temperature of from approximately 25° C to approximately 35° C.

Once applied, the dispensed conductive material 26 is cured. For example, silver ink may be cured at 90 ⁰C for one hour. Other temperatures and curing times may be used based on the properties of the conductive material.

With the conductive material 26 properly applied and cured, the ribbon cable 10 is moved into position relative to a dispensing nozzle 54 of a printhead, as shown in FIG. 5. The dispensing nozzle 54 is aligned with the center of the dispensed conductive material 26 in the respective conductor window 22.

The use of a dispensing nozzle 54 of a printhead allows for the nonconductive or insulative material 30 to be precisely applied to over the conductive material 26 in the respective conductor window 22. In particular, the nonconductive or insulative material 30 can be applied such that the nonconductive or insulative material 30 provides sufficient electrical insulation to protect the micro-junctions of the sensor 12.

Various printing technologies can be used to apply the nonconductive or insulative material 30 through dispensing nozzle 54, including, but not limited to, inkjet printing, contact extrusion dispensing, jet dispensing. In the illustrative embodiment shown, the dispensing nozzle 54 is part of a jet dispensing system. With jet dispensing, the dispensing nozzle or jet 54 hovers over and shoots the nonconductive or insulative material 30 over the conductor window 22 and the conductive material 26 without ever touching the conductive material 26. Therefore, the z-axis motion of dispensing nozzle 54 can be eliminated for printing on uneven or irregularly shaped surfaces of the ribbon cable 10. While various jet dispensing systems may be used, one example is the MDS 3280 dispensing system, available from VERMES Microdispensing GmbH. The dispensing is based on piezoelectric actuator with a unique bayonet fluid box body, which is more suitable for volume production.

The dispensing nozzle 54 may have different configurations and different sizes of an inner channel of the nozzle insert which can affect the dispensing volume. In an illustrative embodiment, the nozzle insert has a conical inner channel, causing the nonconductive or insulative material 30 to accelerate while exiting the dispensing nozzle 54 resulting in less buildup of the nonconductive or insulative material 30 on the dispensing nozzle 54. The size of the tappet tip has an influence on the dispensing energy of the nonconductive or insulative material 30. In one illustrative embodiment, the nozzle insert has an approximately 100 µm nozzle insert and the tappet rod with a tip radius of approximately 7 µm to facilitate the dispensing of the nonconductive or insulative material 30.

In the embodiment shown in FIG. 5, three droplets of the nonconductive or insulative material 30 is dispensed from the dispensing nozzle 54 onto the conductive material 26 and the exposed pairs of conductors 14 in the respective conductor window 22. In other embodiments, one, two or more than three droplets may be applied. The drop size may be between approximately 250 microns to approximately 500 microns in diameter when applied to the conductive material 26 and the exposed pairs of conductors 14 in the respective conductor window 22. In various embodiments, the coating thickness of the nonconductive or insulative material 30 is in the range of approximately 30-50 microns with 3 drops of the nonconductive or insulative material 30 dispensed on the conductive material 26. In various embodiments, it may be desirable to also apply the nonconductive or insulative material 30 to the backside of the cable 10 at the conductor window 22 to provide the electrical insulation.

In one illustrative embodiment, the jet dispensing process setting for the nonconductive or insulative material 30 has: a rise time of from approximately 0.2 ms to approximately 0.3 ms; a fall time of from approximately 0.1 ms to approximately 0.2 ms; and open time of from approximately 0.2 ms to approximately 0.3 ms; a needle lift of from approximately 60% to approximately 80%; a delay of from approximately 0.1 ms to approximately 0.2 ms; a nozzle height of from approximately 1.8 mm to approximately 2 mm; a pressure of from approximately 0.1 MPa to approximately 0.15 MPa; and a temperature of from approximately 25° C to approximately 30° C.

In the illustrative embodiment shown, the nonconductive or insulative material 30 is a UV-curable adhesive. Examples of the UV-curable adhesive are medical device acrylated urethane adhesives from Dymax Corporation. The UV-curable adhesive should contain no nonreactive solvents and cure upon exposure to light.

Once applied, the dispensed nonconductive or insulative material 30 is cured. For example, the nonconductive or insulative material 30 can be cured using a spot UV lamp with approximately a 1.5 W/cm² intensity and approximately 5 seconds to approximately 20 seconds.

While the illustrative embodiment shown in FIGS. 1-6 is directed to a ribbon cable 10 with multiple conductive pairs of cables and multiple sensors 12, other embodiments may be used. For example, FIG. 8 illustrates an alternate embodiment in which a ribbon cable 10' has two wires and a sensor terminating area 20' provided at the end of the ribbon cable 10'. Conductive material (not shown) and insulative material 30' are applied to the exposed conductors sensor terminating area 20' in the same manner as previously described with respect to the ribbon cable 10 with multiple conductive pairs of cables and multiple sensors 12.

In the multi-conductor ribbon cable sensor assembly, conductive material, such as silver ink could be used to form the junctions for the ribbonized MTC sensors. Printing technology, such as jet dispensing, is utilized to apply the conductive material to the laser stripped wire window of the ribbon cable, and UV-curable encapsulant is subsequently coated on the conductive material, with high precision and repeatability. The printed electronics technology combined with precision laser ablation can enable automated reel-to-reel high volume production of the multi-conductor MTC ribbon sensors.

While the invention has been described with reference to a preferred embodiment, it will be understood by those skilled in the art that various changes may be made and equivalents may be substituted for elements thereof without departing from the spirit and scope of the invention as defined in the accompanying claims. One skilled in the art will appreciate that the invention may be used with many modifications of structure, arrangement, proportions, sizes, materials and components and otherwise used in the practice of the invention, which are particularly adapted to specific environments and operative requirements without departing from the principles of the present invention. The presently disclosed embodiments are therefore to be considered in all respects as illustrative and not restrictive, the scope of the invention being defined by the appended claims, and not limited to the foregoing description or embodiments.

## Claims

1. A ribbon cable sensor assembly (2) comprising:
a ribbon cable (10) with respective paired conductors (14);
at least one sensor terminating area (20) positioned on the ribbon cable, the at least one sensor terminating area (20) having insulation removed from respective individual wires (14) of the respective paired conductors to provide exposed individual conductors; and
conductive material (26) applied to respective exposed individual conductors (14) in the at least one sensor terminating area 1(20) to form at least one sensor (12).

2. The ribbon cable sensor assembly as recited in claim 1, wherein the at least one sensor terminating area (20) are multiple sensor terminating areas spaced apart along a longitudinal axis (16) of the ribbon cable (10) and the at least one sensor (12) are multiple sensors positioned in the multiple sensor terminating areas (20) to allow the multiple sensors to sense different regions along the ribbon cable (10).

3. The ribbon cable sensor assembly (2) as recited in claim 1 or 2, wherein the ribbon cable sensor assembly is positioned in a catheter (18).

4. The ribbon cable sensor assembly (2) as recited in claim 2 or 3, wherein each sensor terminating area (20) of the multiple sensor terminating areas has a conductor window (22) having insulation removed from respective individual wires (14).

5. The ribbon cable sensor assembly (2) as recited in claim 4, wherein each conductor window (22) is approximately 150 microns by approximately 400 microns, preferably wherein the multiple sensors (12) are temperature sensors which monitor temperature in a given region along the ribbon cable sensor assembly (2).

6. The ribbon cable sensor assembly (2) as recited in any preceding claim, wherein the conductive material (26) is a conductive metal ink, preferably conductive silver ink (26), wherein the silver ink has approximately 0.3 micron or less diameter silver particles.

7. The ribbon cable sensor assembly (2) as recited in any preceding claim 1, wherein the conductive material (26) has a resistivity of between approximately 0.03 mohm·cm and approximately 5 mohm·cm.

8. The ribbon cable sensor assembly (2) as recited in any preceding claim, wherein the conductive material (26) is diluted with between approximately 0.5% to approximately 3% solvent.

9. The ribbon cable sensor assembly (2) as recited in any preceding claim, wherein the conductive material (26) has a thickness of between approximately 20 microns to approximately 30 microns.

10. The ribbon cable sensor assembly (2) as recited in any preceding claim, wherein the conductive material is cured droplets of between approximately 150 microns to approximately 400 microns in diameter.

11. The ribbon cable sensor assembly (2) as recited in any preceding claim, wherein nonconductive material (30) is applied to at least one sensor terminating area (20) to cover the conductive material (26), wherein the nonconductive material (26) optionally:
is a UV-curable adhesive,
is cured of droplets of between approximately 250 microns to approximately 500 microns in diameter, or
has a thickness of between approximately 30 microns to approximately 50 microns.

12. The ribbon cable sensor assembly (2) as recited in any preceding claim, wherein each sensor (12) is a micro-thermocouple sensor.

13. A method (100) of manufacturing a ribbon cable sensor assembly (2) with multiple sensors (12), the method comprising:
exposing (102) individual conductors (14) of a ribbon cable (10) in a defined area (20) by removing insulation in the defined areas;
applying (104) conductive ink (26) in the defined area (22) on the exposed individual conductors (14) of the cable to form a sensor (12); and
curing (106) the conductive ink (26).

14. The method of manufacturing a ribbon cable sensor assembly (2) as recited in claim 13, further comprising:
applying (108) nonconductive material (30) over the conductive ink (26); and
curing (110) the nonconductive material (30).

15. The method of manufacturing a ribbon cable sensor (2) assembly as recited in claim 14, wherein the conductive ink (26) and the nonconductive ink (30) are applied in droplets by a jet dispensing printing process.

16. The method of manufacturing a ribbon cable sensor assembly (2) as recited in claim 13, 14 or 15, wherein the insulation in the defined areas (20) is removed by laser ablation to form conductive windows (22).
